# EUROPEAN PATENT SPECIFICATION

(11) **EP 4 392 960 B1**
(45) Date of publication and mention of the grant of the patent: **12.03.2025**
(21) Application number: 23703637.1
(22) Date of filing: 03.02.2023
(51) Int. Cl.: G08B 25/14

(54) **DOOR MONITORING SYSTEM**
TÜRÜBERWACHUNGSSYSTEM
SYSTÈME DE SURVEILLANCE DE PORTE

(30) Priority: 07.06.2022 GB 202208351; 11.11.2022 WO PCT/GB2022/052870
(43) Date of publication of application: 03.07.2024
(60) Divisional application: 25155453.1
(73) Proprietor: Kingsway Enterprises (UK) Limited, Swanley, Kent BR8 8TS (GB)
(72) Inventor: HALL, Julian, London Road Swanley Kent BR8 8TS (GB); FOOT, Rod, London Road Swanley Kent BR8 8TS (GB)
(74) Representative: Pfundner, Benjamin Patrick
(86) International application number: PCT/GB2023/050244
(87) International publication number: WO 2023/237848

(56) References cited:
- CN-A- 113 035 345
- US-A- 5 297 252
- US-B2- 10 593 171

## Description

### Technical Field

The present disclosure relates to methods of configuring and using a door monitoring system, in particular in settings where doors contain ligature-detection devices.

### Background

In psychiatric hospitals and prisons, a problem exists that patients and inmates may wish to cause themselves harm using a ligature created by securing a rope or cable around an available anchor point in a room. One solution to this problem is to design room fixtures and fittings such that they do not provide such anchor points. However, in some cases this is difficult or impossible. An example of this is door fittings. Individuals may try to create a ligature by securing a rope or cable around an edge of a door leaf.

One solution is to attach a ligature-detection device, typically a switch, to an edge (e.g. top edge) of a door leaf. When a ligature is secured around the device and pressure is applied, the switch is caused to close, thereby completing or breaking an electrical circuit and activating an alarm. Accordingly, while the door leaf may itself remain a potential ligature hazard, safety is nonetheless improved because a nearby prison officer or healthcare professional is alerted when an individual attempts to secure a ligature around the door leaf.

Unfortunately, problems in preventing self-harm in this manner remain. In particular, it may take staff a long time to determine the source of an alarm and find the correct room so that they may administer assistance. Additionally, ligature-detection devices are vulnerable to tampering and signal loss. There is often a significant delay between a ligature-detection device being tampered with or rendered ineffectual and staff becoming aware of this. This 'down-time' in the ligature-detection mechanism undermines the security of inmates and patients.

Further issues arise as a result of ligature-detection devices and alarm systems being administered by different parties. Often, a third party system is used to provide alarm functionality. This third party system must therefore 'plug-in' or otherwise interface reliably with the ligature-detection devices and other door sensors. However, this mechanism is often unreliable and the presence of potentially multiple third party systems interfacing with the monitored doors is in any case undesirable, because this can cause a lack of continuity between alarm systems and cause confusion for staff members. This can hinder prompt assistance being provided in an emergency. Such a system also makes configuration of a door monitoring system difficult. The use of 'plug-in' third party systems also presents difficulties in that it is then typically the third party that records alarm data (if any), as opposed to the institution where the doors and ligature-detection devices are provided. This results in unnecessary and unwanted complication in obtaining alarm records or other door-related data from the third party, as may be required for auditing or security purposes.

As can be seen, existing mechanisms for monitoring doors comprising ligature-detection devices suffer from significant drawbacks. It would be advantageous to provide systems and methods which address one or more of these problems, in isolation or in combination.

US5297252A describes a color graphics terminal for monitoring a system in which a graphics monitor is connected to a computer. At least two separate graphics windows are capable of representing simultaneously two geographically, hierarchically related graphic displays of the system, with an arrangement for controlling the computer to automatically display on the graphics monitor the related graphic displays in response to certain inputs received by the computer.

US10593171B2 describes an anti-ligature alarm device, comprises a sensing unit and a control unit. The sensing unit detects an external force when the external force is applied to the sensing unit and also transmits a signal indicating an alarm condition. The control unit comprises a receiver which receives the signal on detection of the external force. The sensing unit also includes a power source and a controller and the sensing unit and control unit are wirelessly paired to each other. The sensing unit also comprises a boost module which maintains a voltage input from the power source to the controller. The control unit is wirelessly networked to an alert device and the anti-ligature alarm device further includes a pairing device which is used to pair the sensing unit and the control unit.

CN113035345A describes a mattress with vital sign monitoring and a monitoring system thereof.

### Overview

This overview introduces concepts that are described in more detail in the detailed description. It should not be used to identify essential features of the claimed subject matter, nor to limit the scope of the claimed subject matter.

According to one aspect of the present disclosure, there is provided a computer-implemented method of claim 1.

Such a method may be used for a door monitoring system in a prison or psychiatric hospital to address at least some of the problems outlined above. In particular, this method enables a door monitoring system to be intuitively configured such that the correct doors are selected for monitoring. This can be achieved easily and intuitively because users who provide the selection are presented with an intuitive floor plan display showing the location of the various candidate doors. Users then simply select the doors for monitoring, for example by direct interaction with the displayed floor plan.

By placing the candidate doors in context in a displayed floor plan, the layout and location of the doors is more easily understood. This enables the door monitoring system to be configured more quickly and intuitively and provides a significant improvement over, for example, systems which simply require users to enter a list of door numbers for monitoring. Such a system is undesirable because a user may not know where doors are located, either in absolute terms or relative to other doors. A user may also not know the precise door number that is to be monitored. This makes setup time consuming and difficult as the precise locations and numbers of different doors must be determined. The presently disclosed mechanism overcomes these drawbacks and allows the door monitoring system to be configured more intuitively, quickly and effectively.

The user input may comprise use of a drag-and-drop functionality to select a door for monitoring. This provides a yet further intuitive mechanism for selecting a door. For example, the drag-and-drop functionality may comprise dragging and dropping an icon over a displayed door on the floor plan to select that door for monitoring. This provides a quick and easily understandable mechanism for selecting which door(s) to monitor, allowing the door monitoring system to be configured more quickly and easily.

The user input may associate an output of the selected door with an IP address for receiving status updates from the selected door. In other words, selection of a door through the user input may trigger the system to identify a door associated with the location on the graphical interface at which the user selection has taken place. That identified door may then be paired or associated with a particular IP address. Once this connection is established, outputs from the door (such as relating to alarms or faults) can be transmitted to the IP address. This data can then be accessed and processed by a computing apparatus, as described in further detail herein.

As can be seen, this approach provides a mechanism by which a simple user input (such as dragging-and-dropping an icon onto a door on a displayed floor plan) can configure the back-end system to obtain information from the selected door. This avoids the need for the user to do any manual coding or such like to initiate a connection with a selected door. While linking to an IP address provides one mechanism, it will be appreciated that other approaches for obtaining data from a door following selection will be apparent and can be used in the context of the methods and systems disclosed herein.

Based on the user input, a hardwired connection is established between the selected door and a computing apparatus of the door monitoring system. In particular, once a door has been selected for monitoring, data from that door may be obtained via an end-to-end, hardwired connection via, for example, ethernet cables or other wired connections. Such a connection is more reliable than a wireless connection. For example, a wireless connection is vulnerable to drop-out or to the use of Wi-Fi blockers or similar technology. Wireless devices are typically also battery powered, meaning they may fail if batteries are not replaced regularly. All of these factors increase the risk that part of a door monitoring system, particularly a ligature-detection device, may be inoperable at some point, placing inmates or patients at risk because changes of door state (for example detection of a ligature) may not be recorded or received by the door monitoring system. By using a hardwired connection instead, these risks are mitigated. Use of a hardwired connection also provides a more secure connection for sensitive data, as may often be transferred in setting such as hospitals, psychiatric wards and prisons.

Monitoring a status of the selected door may comprise: determining whether a status of the selected door has changed; and responsive to determining that a status of the selected door has changed, modifying the manner in which the selected door is presented on the displayed floor plan.

In this manner, changes in door state (for example initiation of an alarm associated with a ligature-detection device on a door) can be intuitively displayed to a user, such as a psychiatric nurse or prison guard. Various changes in how the door is presented on the display can be used. For example, the door may be visually emphasised through a flashing icon. Alternatively, the door or associated icon may change colour. The door may also be added to a displayed list of doors associated with the change of state that has taken place. For example, if the door state has changed from "closed" to "open", an icon provided at the door on the displayed floor plan may begin to flash, and the door may appear on a list of open doors provided on the display. Similar or alternative visual enhancements may occur if the door state changes in respect of a ligature-detection device, such as if a ligature is detected or if a fault with the ligature-detection device is identified. Where a user drags-and-drops an icon onto a door during the configuration phase, the visual appearance of that icon may change when a state of the associated door changes.

Monitoring a status of the selected door may comprise transmitting a status update request to the selected door to determine at least one of: whether the selected door is open or closed; whether the ligature-detection device of the selected door has been triggered; whether the status of the selected door or of the or ligature-detection device can be accurately determined; and whether the selected door has responded to the status update request.

In this manner, status changes at the door can be quickly identified. In some examples, status update requests are sent periodically to each door. For example, the door(s) selected for monitoring may be polled or 'pinged' at intervals for status updates. Any changes can be recorded and identified to the user via the displayed floor plan as described herein. Similarly, failure by a door to respond to a status update request can be taken to represent a fault at the door (or the door's ligature-detection device) and can be similarly flagged to the user via the visual display.

The method may further comprise: if a status of the selected door changes, providing an auditory indication of the change of status. An auditory indication provided at, for example, the display may further assist in catching a staff member's attention. This may be particularly useful when the display is not under constant surveillance. For example, a display may be provided in a hallway in a prison or psychiatric ward. When a door state changes, an auditory and/or visual alert or alarm may be sounded at the display, thereby drawing staff members' attention to the display so that they can locate the problem.

If the ligature-detection device of the selected door is triggered (activated, depressed or otherwise engaged), the method may comprise determining a duration for which the ligature-detection device has been triggered. In other words, the method may comprise determining how long the ligature detection device has been triggered (or activated) for. The nature of any auditory or visual alerts can then be based on this determined duration.

For example, the method may comprise providing an auditory indication that the ligature-detection device of the selected door has been triggered, wherein the auditory indication is based on the duration for which the ligature-detection device has been triggered. In one example, the auditory indication may be configured such that: when the ligature-detection device has been triggered for less than a threshold duration, a first type of auditory indication is sounded; and when the ligature-detection device has been triggered for more than a threshold duration, a second (different) type of auditory indication is sounded.

In one example, if the ligature-detection device is triggered for less than a given threshold duration, for example seven seconds, then a first type of auditory alarm is sounded. The first type of auditory alarm may be relatively short in duration, may have a relatively low volume and may have a distinct tone indicative of a brief triggering of the ligature-detection device. Such an indication may be considered as a "pre-alarm". An example of a pre-alarm may be a single, low-volume tone that is sounded once.

If the triggering continues beyond the threshold duration, then a second type of auditory alarm may be sounded. The second auditory alarm may be relatively long in duration (or be continuous/constant), may have a relatively high volume and may have a distinct tone indicative of a prolonged triggering of the device. Such an indication may be considered as a "full-alarm". An example of a full alarm may be a high-volume tone that is sounded continuously.

This functionality is advantageous in that a brief triggering of the ligature-detection device, which may be benign, can be distinguished easily and quickly from a prolonged triggering event, which is more likely to represent a potentially life-threatening ligature-attachment event. It will be apparent that a variety of tonal effects may be applied to ensure that a full-alarm is perceived as more urgent than a pre-alarm and is more likely to catch the attention of a member of staff at the facility.

Pre-alarms and full-alarms can also be distinguished visually. In particular, the method may further comprise modifying the manner in which the selected door is presented on the displayed floor plan to indicate that the ligature-detection device of the selected door has been triggered. The manner in which the selected door is presented on the displayed floor plan may be modified based on the duration for which the ligature-detection device has been triggered.

In one example, the manner in which the selected door is presented on the displayed floor plan is modified such that: when the ligature-detection device has been triggered for less than a threshold duration, a first type of visual modification is applied; and when the ligature-detection device has been triggered for more than a threshold duration, a second (different) type of visual modification is applied.

In other words, the pre-alarm and full-alarm functionality described above in the context of an auditory indication may be implemented also (or alternatively) with visual equivalents. Accordingly, in some examples, when the trigger threshold duration has not yet been exceeded, a first type of visual modification may be applied to the door in question. For example, the door (or an icon associated with it) may flash (or pulse) slowly or may change colour to a first colour (e.g. orange). If the trigger threshold is exceeded, a second type of visual modification may be applied. In particular, the nature of the visual modification may change to become more eye-catching and to convey more easily the severity of the situation. For example, the door (or an icon associated with it) may flash (or pulse) rapidly or may change colour to a second colour (e.g. red). As in the case of the auditory indications mentioned above, such visual indicators can quickly and effectively convey the severity of a ligature-detection event.

For both auditory and visual indicators, the threshold duration between the "pre-alarm" and "post-alarm" functionality may be at least 2 seconds. The present inventors have identified that setting the threshold duration at or above this duration minimises the number of false alarms, in other words full-alarms that are caused by accidental or benign triggering of the ligature-detection device. This is because benign or accidental triggering events typically last less than 2 seconds.

Similarly, the threshold duration between the "pre-alarm" and "post-alarm" functionality may be at most 7 seconds. The present inventors have identified that setting the threshold duration at or below this duration ensures that actual ligature events result in a full alarm sufficiently quickly to enable a swift enough response. If the threshold is set higher than 7 seconds, then staff will be delayed to a dangerous extent in responding to a ligature attachment event.

Hence, the threshold duration is advantageously between 2 and 7 seconds. For example, the threshold duration may be 2 seconds, 3 seconds, 4 seconds, 5 seconds, 6 seconds or 7 seconds.

The method may further comprise configuring the door monitoring system to additionally monitor the status of one or more staff alarm devices and/or one or more patient call devices. This can be achieved in any suitable way, for example the door monitoring system and the staff alarm devices can be paired (e.g. using a suitable mechanism such as Bluetooth^{®}) or monitored via a hard-wired connection. If the staff alarm device or patient call device is portable, the door monitoring system may advantageously be configured to monitor the location of the device through suitable technology such as Bluetooth^{®}, GPS or other similar technology to enable real-time or periodic location tracking.

The method may further comprise configuring the door monitoring system to additionally monitor the status of one or more door observation panels. In particular, the system may monitor when the door observation panels (which are used to enable staff to view into a patient or inmate's room) are opened, indicating that staff are checking in on patients/inmates.

The method may further comprise configuring the door monitoring system to additionally monitor the status of one or more vital sign monitoring devices. Such vital sign monitoring devices include wearables or sensors that may be provided to monitor vital signs of staff and/or room occupants e.g. patients/inmates.

According to a further aspect of the present disclosure, a computer-implemented method of identifying, by a door monitoring system, a change of status at a monitored door is disclosed. The method comprises: displaying a floor plan of a building comprising one or more monitored doors, each monitored door comprising a ligature-detection device; identifying that a status of a first monitored door of the one or more monitored doors has changed; and indicating that a status of the first monitored door has changed by modifying the manner in which the first monitored door is presented on the displayed floor plan.

Such a method provides an intuitive and clear mechanism for alerting staff members where a door status change has occurred. In particular, staff members are provided with the geographical context of an alert or alarm because the door in question is shown on a displayed floor plan and the appearance of the door is modified to make it stand out from other doors. This allows staff members to locate the required door and provide assistance to that location more quickly and easily, improving response times and patient/inmate safety.

Identifying that a status of the first monitored door has changed may comprise determining that the first monitored door has been opened or closed. In other words, the door monitoring system can provide an intuitive and clear indication when monitored doors are opened and closed, aiding staff members in checking for irregularities and ensuring patient/inmate safety. Identifying that a status of the first monitored door has changed may comprise determining that the ligature-detection device has been triggered. In other words, the door monitoring system can provide an intuitive and clear indication that a ligature-detection device of a particular door has been triggered. This allows staff members to respond more quickly in the event that a person is attempting to self-harm using a ligature.

As above, if the ligature-detection device of the first monitored door is triggered, the method may comprise determining a duration for which the ligature-detection device has been triggered. Also as above, the method may comprise providing an auditory indication that the ligature-detection device of the first monitored door has been triggered, wherein the auditory indication is based on the duration for which the ligature-detection device has been triggered.

As above, the auditory indication may be configured such that: when the ligature-detection device has been triggered for less than a threshold duration, a first type of auditory indication is sounded; and when the ligature-detection device has been triggered for more than a threshold duration, a second type of auditory indication is sounded. The advantages of providing a distinct "pre-alarm" phase which precedes a "full alarm", as well as example implementations of this approach, are as described above.

As also described above, the method may further comprise modifying the manner in which the first monitored door is presented on the displayed floor plan to indicate that the ligature-detection device of the selected door has been triggered, wherein the manner in which the first monitored door is presented on the displayed floor plan is modified based on the duration for which the ligature-detection device has been triggered.

In one example, similar to the example described above, the manner in which the first monitored door is presented on the displayed floor plan is modified such that: when the ligature-detection device has been triggered for less than a threshold duration, a first type of visual modification is applied; and when the ligature-detection device has been triggered for more than a threshold duration, a second type of visual modification is applied. The advantages and example implementations of this approach are as described above.

As above, the threshold duration may be between 2 and 7 seconds (inclusive) which ensures an effective balance between reducing false alarms and ensuring rapid response to genuine ligature-detection events.

Identifying that a status of the first monitored door has changed may comprise determining that a fault with the ligature-detection device has been detected. In other words, the door monitoring system can provide an intuitive and clear indication that a ligature-detection device of a particular door is no longer working properly. This allows staff members to respond and fix the fault more quickly, reducing 'down-time' in ligature-detection devices and improving patient/inmate safety.

Identifying that a status of the first monitored door has changed may comprise determining that the state of the first monitored door or of the ligature-detection device can no longer be accurately determined. In other words, the door monitoring system can provide an intuitive and clear indication that the status of a door or ligature-detection device may be unknown or at issue. This again allows staff members to respond and fix any potential faults more quickly, reducing 'down-time' in ligature-detection devices and door sensors and improving patient/inmate safety.

Identifying that a status of the first monitored door has changed may comprise determining that the first monitored door or ligature-detection device has not responded to a status update request. In other words, the door monitoring system can provide an intuitive and clear indication that the status of a door or ligature-detection device may be unknown or at issue, as may occur if a connection to the door is faulty or has been tampered with. This also allows staff members to investigate and fix any potential faults more quickly, reducing 'down-time' in ligature-detection devices and door sensors and improving patient/inmate safety.

Modifying the manner in which the first monitored door is presented on the displayed floor plan may comprise at least one of: visually emphasising the first monitored door; changing a colour associated with the first monitored door; and adding the first monitored door to a displayed list of doors associated with having an alarm, having a fault, and/or being opened.

As already discussed above, this functionality enables changes in door state (for example triggering of a ligature-detection device on a door) to be intuitively displayed to a user, such as a psychiatric nurse or prison guard. The change of state can be displayed in an eye-catching manner, so as to capture the attention of a staff member who may be in proximity of the display. This is important because displays may not be monitored at all times; rather, staff members may be walking past or working near the displays. Accordingly, providing an intuitive mechanism for capturing staff members' attention at the display when a door state change is detected is beneficial in increasing response times.

As also noted above, the modification in how the first monitored door is presented on the displayed floor plan may differ based on which aspect of the monitored door's status has changed. For example, a change in state relating to whether the door is open or closed may be associated with a first visual modification. Similarly, a change in state relating to whether the ligature-detection device of the door has been triggered may be associated with a second visual modification. A change in state relating to whether a fault has been detected at the door or ligature-detection device may be associated with a third visual modification. The visual modification relating to a triggered ligature-detection device may be more extreme, persistent or eye-catching than other visual modifications, given the urgent nature of such an event.

The method may further comprise: logging, in a database, an indication that a status of the first monitored door has changed. In this manner, the door monitoring system can record door status changes. In this manner, door status monitoring, alarm/alert provision and event logging are all performed by the same, single door monitoring system. This provides improved reliability and accessibility of data compared to existing mechanisms where all three aspects are provided by different parties and distinct systems.

The method may further comprise: responsive to receiving the indication that a status of the first monitored door has changed, causing an auditory indication (e.g. an auditory alarm) to be sounded. As described above, an auditory indication may further assist in capturing staff members' attention at the display or elsewhere.

In some examples, an auditory indication is only used when a ligature-detection device is triggered, given the urgency of such an event. Alternatively, the auditory indication may differ based on which aspect of the monitored door's status has changed. Accordingly, a ligature-detection event may result in a louder, more persistent or more extreme or unusual sound being played than a door opening/closing event or a fault detection event.

The method may further comprise periodically polling the one or more monitored doors for an indication of their respective statuses. This allows status information to be regularly updated for each monitored door. By polling regularly (for example every few seconds), door statuses can be updated quickly to allow rapid response to dangerous events.

Each monitored door may be monitored via a hardwired connection between the respective door and the other components of the door monitoring system, such as a computing apparatus. As discussed above, this may provide a more reliable and secure connection to each door.

The method may further comprise identifying that a status of a monitored staff alarm device or patient call device has changed. For example, the system may identify that a button or trigger on the device has been actuated. Responsive to this identification, the method may comprise indicating that a status of the monitored staff alarm device or patient call device has changed by modifying the manner in which the staff alarm device is presented on the displayed floor plan. For example, an icon associated with the device may appear or change appearance on the display. A visual and/or auditory indication may be used to draw attention to the event. Preferably, if the location of the triggered device is known to the system, then any visual indication of the event is shown at the correct location on the displayed floor plan so as to immediately indicate the location of the event. Staff attack alarm events and patient call events can be distinguished visually and/or auditorily from other event types so that staff can quickly and intuitively determine the type of event that is occurring and respond appropriately in an efficient manner. Such event data can also be recorded and stored for future auditing purposes, which is important in healthcare and prison settings to ensure quality of service.

The method may further comprise identifying that a monitored door observation panel has been opened and recording in memory an indication that the monitored door observation panel has been opened. For example, the date, time and location of the opened door observation panel can be recorded in memory (e.g. in a database) for future auditing. This ensures that a record of when patients/inmates are being observed can be accurately kept in a fraud-resistant manner that is difficult to spoof. This ensures quality of care for patient/inmates and can be used to easily identify malpractice (e.g. if patients are not being observed frequently enough).

The method may further comprise identifying that a status of a monitored vital sign monitoring device has changed and indicating that a status of the monitored vital sign monitoring device has changed by modifying the manner in which the vital sign monitoring device is presented on the displayed floor plan. For example, an icon associated with the device may appear or change appearance on the display. In one example, a raised heartrate detected by a vital sign monitoring device may result in a change in the display. Changes of statuses in vital sign monitoring devices can be presented in any suitable manner as described above in relation to other devices, for example using a suitable visual and/or auditory indication. Preferably the location of the device is also shown on the display in the context of the displayed floor plan to enable rapid response by staff.

According to a further aspect of the present disclosure, a computer apparatus configured to perform any of the methods described herein is disclosed.

According to a further aspect of the present disclosure, a computer-readable medium comprising instructions which, when executed by a computer, cause the computer to carry out any of the methods described herein is disclosed.

According to a further aspect of the present disclosure, a computer program comprising instructions which, when the program is executed by a computer, cause the computer to carry out any of the methods described herein is disclosed.

### Brief Description of the Figures

Illustrative implementations of the present disclosure will now be described, by way of example only, with reference to the drawings. In the drawings:
Figure 1 shows a door monitoring system according to an example implementation of the present disclosure;
Figure 2 shows an example implementation of a hardwired door monitoring system according to the present disclosure;
Figure 3 shows an example graphical user interface for implementing the methods of the present disclosure;
Figure 4 shows a method of configuring a door monitoring system according to the present disclosure;
Figure 5 shows a method of identifying, by a door monitoring system, a change of status at a monitored door according to the present disclosure; and
Figure 6 shows an example computer apparatus that can be used to implement the methods of the present disclosure.

Throughout the description and the drawings, like reference numerals refer to like features.

### Detailed description

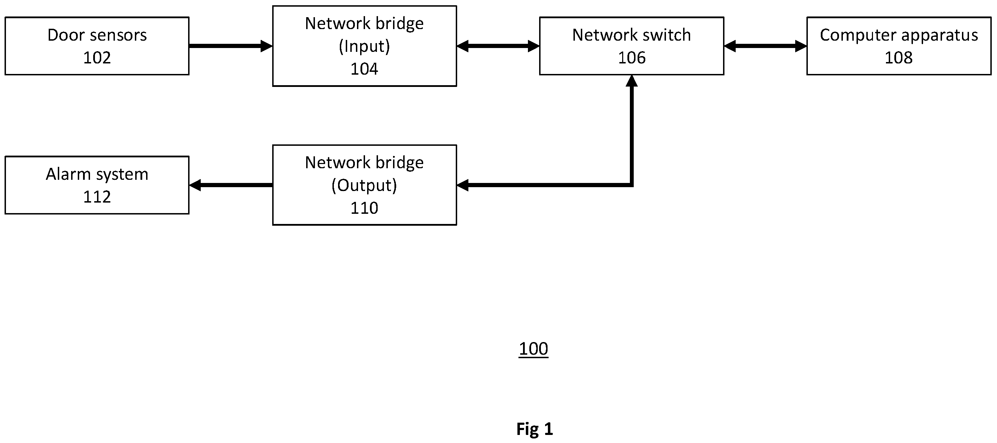

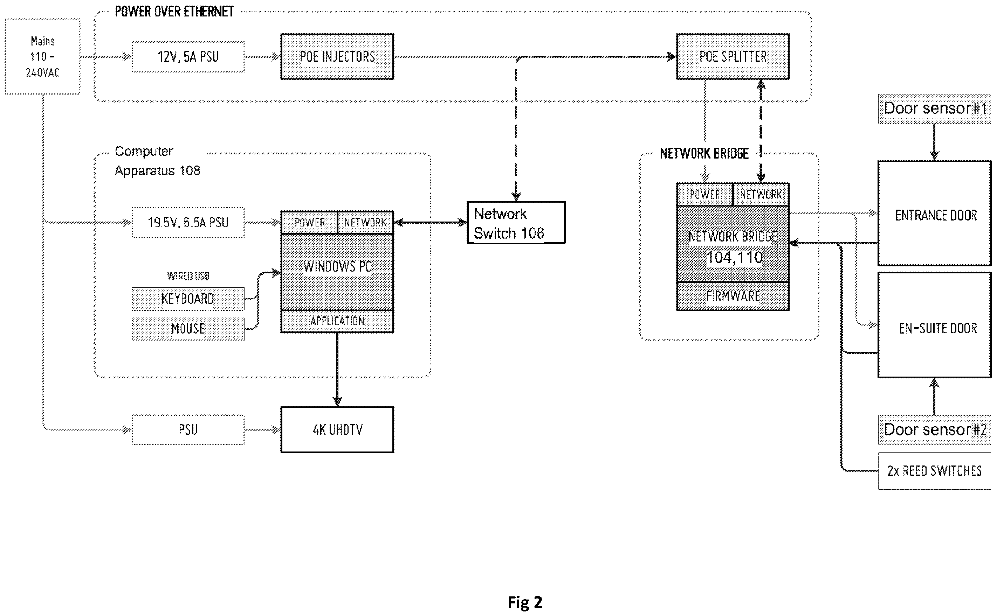

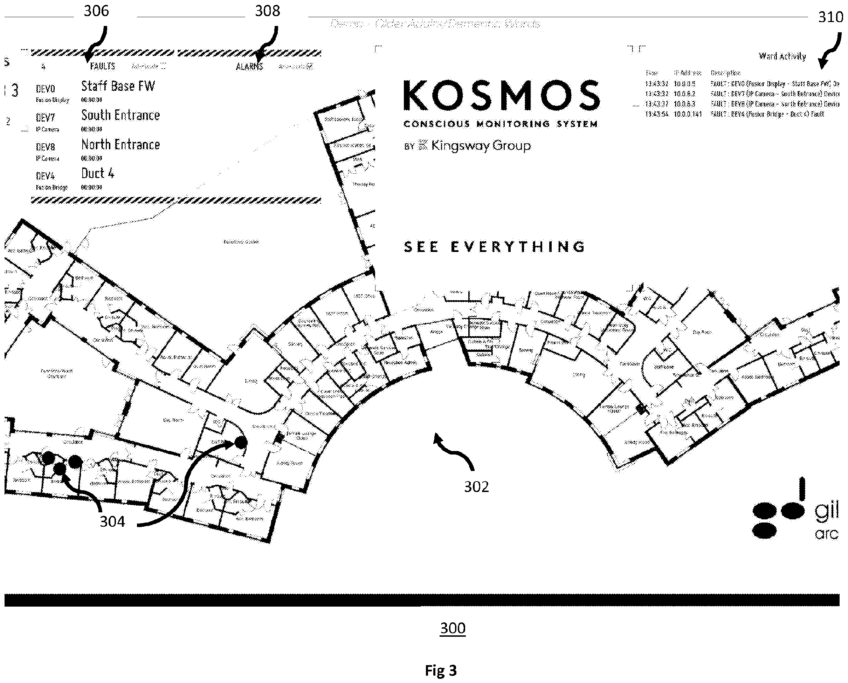

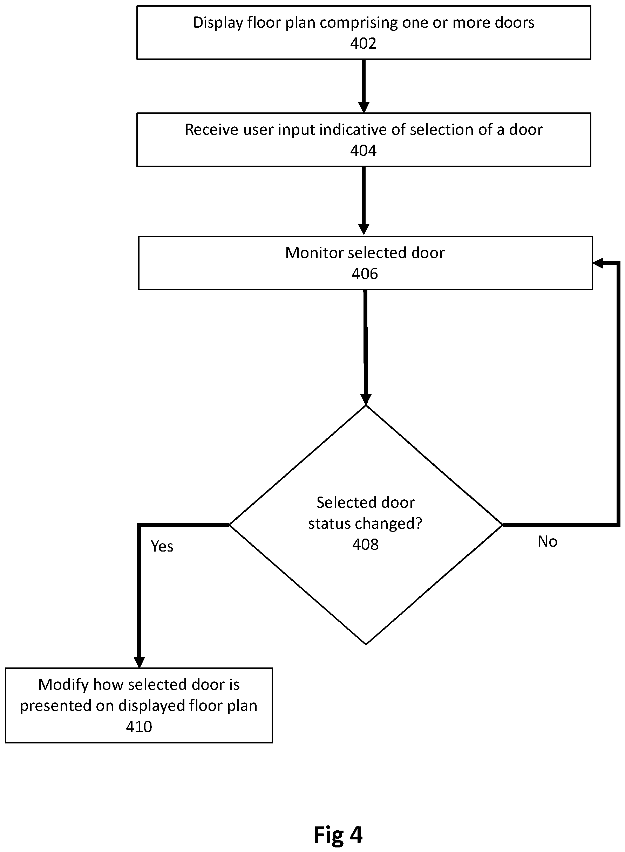

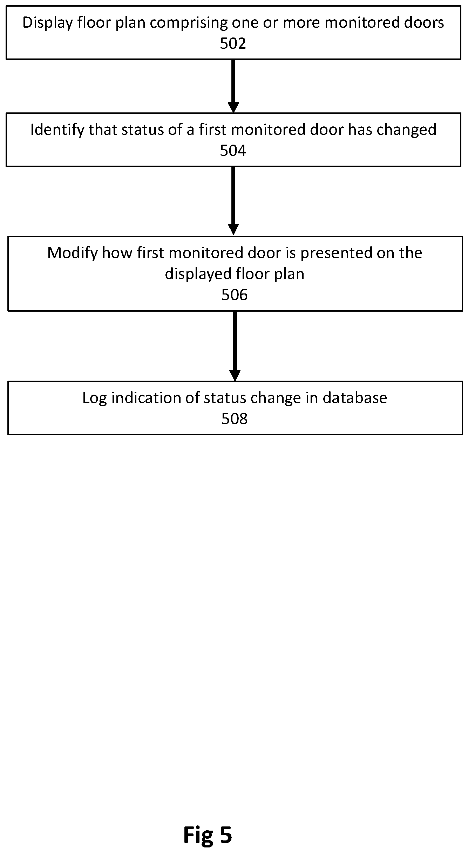

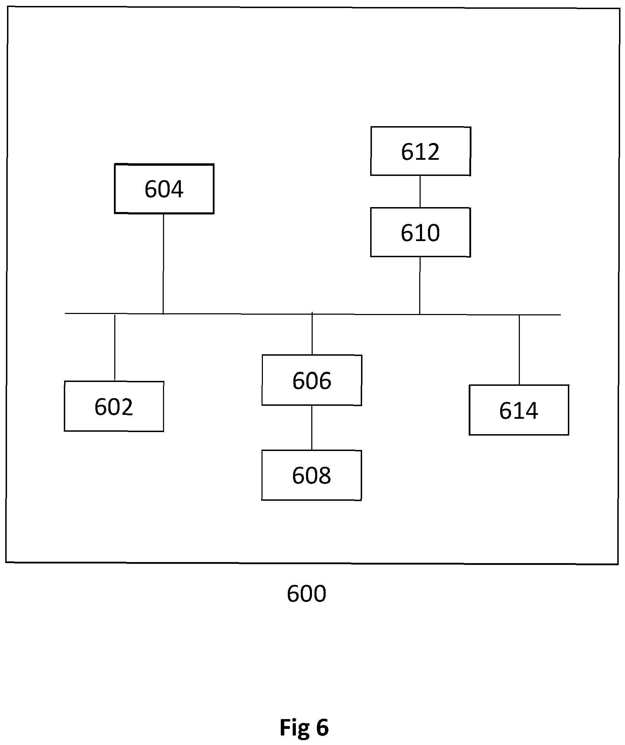

This detailed description describes, with reference to Figure 1, a door monitoring system that can be used to implement the methods disclosed herein. A hardwired implementation of the disclosed door monitoring system is discussed with reference to Figure 2. An example graphical user interface which can be used to implement the disclosed methods is described in reference to Figure 3. Methods of configuring and using the disclosed door monitoring system are discussed in relation to Figures 4 and 5 respectively. Finally, a computer apparatus of the type which form part of the door monitoring system and may be used to implement the methods disclosed herein is described with reference to Figure 6.

The methods disclosed herein relate generally to door monitoring systems and methods for configuring and using the same. The disclosed methods and systems provide a more intuitive and reliable mechanism for both configuring and using a door monitoring system in an environment containing doors having ligature-detection devices. The safety of the persons housed in the monitored building is thereby improved.

Turning first to Figure 1, an example implementation of a door monitoring system 100 is schematically shown. Such a door monitoring system may be used to enable monitoring of doors in a location such as a prison or psychiatric hospital.

Door monitoring system 100 comprises one or more door sensors 102. These door sensors 102 comprise a ligature-detection device configured to identify when a ligature may be being tied or secured around the door. In some contexts, such ligature-detection devices may be referred to as 'door alarms'. A ligature in this context means a rope, cable, noose or other binding which may be used by a person to harm themselves once secured to a door.

A variety of ligature-detection devices are available and can be used in the context of the present disclosure. For example, United Kingdom patent applications GB1916899.6 and GB1918884.6, both in the name of Kingsway Enterprises (UK) Limited, disclose example ligature-detection devices which use ribbon switches to detect pressure applied by a potential ligature secured to the door. The disclosures of these references are hereby incorporated in full. Similarly, GB2109187.1, also in the name of Kingsway Enterprises (UK) Limited, discloses an inductive ligature-detection device which uses inductance to detect a potential ligature. The disclosure of this reference is also hereby incorporated in full. Other examples of ligature-detection devices will be known to the skilled person and can be used in the systems and methods of the present disclosure.

As well as ligature-detection devices, door sensors 102 may comprise additional sensors or devices. For example, door sensors 102 may include a sensor to determine whether a door is open or closed. A variety of sensors which may be used for this purpose, such as pressure or inductive sensors, will be apparent to the skilled reader. Similarly, door sensors 102 may comprise devices or sensors for determining if a fault has occurred with one or more other sensors, such as a ligature-detection device. In one example, and end-of-line resistor is provided. Such an end-of-line resistor enables faults or errors with other door sensors 102, such as a ligature-detection device, to be identified and alerted to a user, for example at computer apparatus 108 as will be described in further detail below.

Accordingly, throughout this disclosure reference to transmitting signals to, or receiving signals from, a monitored door should be construed as involving the transmittal or receiving of signals to/from door sensors 102 and/or associated components included in the door or surrounding environment (e.g. doorframe) and used to monitor a status of the door.

In the example implementation of Figure 1, the door monitoring system 100 is configured as a network, such as a TCP/IP network. Accordingly, an input network bridge 104 receives data from door sensors 102 and provides the data to the remainder of the door monitoring system network, in particular computer apparatus 108. Input network bridge 104 thus allows the outputs of door sensors 102 to be accessible over the network. Similarly, an output network bridge 110 facilitates communication from the network, in particular computer apparatus 108, with alarm system 112. Accordingly, output network bridge 110 enables the wider network, and particularly computer apparatus 108, to sound alarms and activate visual indicators in response to various predetermined conditions, as will be described in further detail below.

As explained in more detail in relation to Figure 2, the door monitoring system 100 network can be hardwired. The input network bridge 104 and output network bridge 110 can be powered locally or via the network, such as through power over ethernet (PoE) facilities using ready-made PoE splitters. In an example, power is fed directly to door sensors 102 via Cat-6 cables. Similarly, existing LAN switches, routers, firewalls and cables can be used to provide a network backbone over which data and power can be transferred. This provides a more reliable means of powering door sensors 102 and other components of the door monitoring system 100 than wireless networks.

Input network bridge 104 and output network bridge 110 may be associated with a particular computer apparatus 108 or display. In this case, the network bridges 104,110 may monitor their connection to their nominated display or computer apparatus 108 and seek out other displays to report a loss of connection, should such a loss occur.

Door monitoring system 100 also includes, in the example of Figure 1, a network switch 106. Network switch 106 further facilitates connection of door sensors 102 and alarm system 112 to a wider network, such as computer apparatus 108. The network can be simple or of any complexity depending on the site requirements. For example, networks can extend to other sites and monitoring stations and this is facilitated by network switch 106. The architecture and distribution of network bridges and network switches will vary according to the implementation, as will be appreciated by a skilled reader.

Door monitoring system 100 also includes computer apparatus 108. It will be appreciated that there may be more than one computer apparatus 108 on the network; for simplicity, only a single apparatus is shown. Computer apparatus 108 is configured to perform the methods described herein. In particular, computer apparatus 108 runs a suitable software application which displays a floor plan or site plan, showing the location of rooms and doors in the building being monitored. As will be described in further detail below, computer apparatus 108 can be interfaced with by a user to configure door monitoring system 100 to monitor specific doors, in particular to monitor door sensors 102 of specific doors. Further, computer apparatus 108 can provide a display which dynamically shows alarm and fault events associated with monitored doors (specifically door sensors 102 of said specific doors) in real time.

Computer apparatus 108 maintains contact with each network bridge to help detect faults in the network. Computer apparatus 108 can also log door event data such as status changes in a database, to facilitate event tracking and data visibility.

Door monitoring system 100 is configured to detect the disconnection of any sensors, bridges, switches or computer apparatuses. Powered devices are configured to report loss of power, either by a signal line or by a failure to respond to a status update request transmitted by computer apparatus 108.

As noted above, door monitoring system 100 and in particular computer apparatus 108 are configured to perform the methods described in further detail in this disclosure. In particular, door monitoring system 100 can detect, via door sensors 102, that: a monitored door has been opened or closed; that a ligature-detection device associated with a monitored door has been triggered; and/or that a fault with a door sensor (such as a ligature-detection device) has been identified. Such events or 'door state changes' are communicated via the network (in particular input network bridge 104 and network switch 106) to computer apparatus 108. The change of state of the door is displayed visually at a display of computer apparatus 108 in order to draw the attention of a staff member, as described in detail with reference to Figures 3 and 5 below. In the example of Figure 1, responsive to the identified change of state, computer apparatus 108 also transmits a notification (via network switch 106 and output network bridge 110) to alarm system 112. This causes alarms situated around the building to be sounded as appropriate based on the change of state. In an alternative arrangement alarm system 112 may be omitted and the only alarms sounded may be those at the computer apparatus 108 and/or the display linked to the computer apparatus 108.

As described above, providing door monitoring system 100 as a hardwired system provides numerous benefits. In particular, such a connection is more reliable than a wireless connection. For example, a wireless connection is vulnerable to drop-out or to the use of Wi-Fi blockers or similar technology. Wireless devices are typically also battery powered, meaning they may fail if batteries are not replaced regularly. All of these factors increase the risk that a door monitoring system, particularly a ligature-detection device, may be inoperable at some point, placing inmates or patients at risk because changes of door state (for example an alarm) may not be recorded or received by the door monitoring system. By using a hardwired connection instead, these risks are avoided. Use of a hardwired connection also provides a more secure connection for sensitive data, as may often be transferred in settings such as hospitals, psychiatric wards and prisons.

An example implementation of a hardwired network of the sort described with reference to Figure 1 is shown in subsequent Figure 2. It will be appreciated that this figure merely shows one schematic arrangement of how the network may be configured and should not be considered as being limiting.

As can be seen, a hardwired connection is provided between all components of the door monitoring system 100. In particular, power over ethernet (PoE) functionality is used to provide power and data between devices. An example computer apparatus 108 is shown in greater detail, comprising power and network connections, a Windows^{™} operating system running an application to enable door monitoring functionality, and input keyboard and mouse devices. The computer apparatus 108 in this example is powered via a power supply unit (PSU). It will be appreciated that other configurations of computer apparatus 108 can be provided, and the displayed example is not intended to be limiting.

In the example shown, computer apparatus 108 is connected to a 4K resolution UHD monitor (e.g. television), on which the floor plan, doors and alerts are shown in the manner discussed more fully below in relation to Figures 3-5. This monitor may be provided, for example, in a staff room or in a corridor of the building in which doors are being monitored, such that there is a high likelihood that a member of staff is nearby to the screen at all times in order to respond to alerts and alarms. It will again be appreciated that, in some examples, a display is additionally or alternatively provided as part of computer apparatus 108 itself. The computer apparatus is connected via network switch 106 and input/output network bridges 104, 110 to two doors. The network bridge is in this example shown as a single schematic entity, for simplicity.

In the example shown, each door has a respective sensor comprising a reed switch. These equate to door sensors 102 described in relation to Figure 1 and, in the present example, represent the sensors of ligature-detection devices comprised in each respective door. It will be appreciated that additional sensors may be provided for each door, as described in relation to Figure 1. As described above, computer apparatus 108 can periodically poll door sensors #1 and #2 to receive status updates. If a status changes, for example if door sensor #1 or #2 is activated or triggered, then this can be displayed on the television connected to computer apparatus 108. Alarm system 112 is not shown in Figure 2, however it will be appreciated that any number of alarms can be included in the wired network and can be provided throughout the building which is being monitored.

Turning now to Figure 3, a graphical user interface (GUI) 300 of the sort which can be displayed by computer apparatus 108 to implement the disclosed methods is shown. Specifically, this interface can be displayed on one or more displays or screens connected to computer apparatus 108, such as the television shown in Figure 2. It will be appreciated that multiple screens each showing GUI 300 may be provided and situated at appropriate locations throughout the building being monitored.

GUI 300 shows a floor plan 302 of the building in which the door monitoring system 100 is provided. As can be seen, floor plan 302 shows the rooms of the building and any associated doors.

During a setup phase (described in greater detail below in relation to Figure 4), GUI 300 may be used to configure the system to monitor one or more specific doors. In the example of Figure 3, four doors have been selected for monitoring. These are visually identified by icons 304 which have been placed over the doors selected to be monitored. It will be appreciated that icons 304 can in practice take any suitable form and may be coloured and/or dynamic. The system can also be configured to monitor other types of device, such as staff alarms, patient call alarms, door observation panels and vital sign monitoring devices, as described more fully below.

A fault list 306 lists faults that have recently been identified by the door monitoring system 100 and is displayed as part of the GUI 300. This provides an easily understandable indication of what faults have occurred at which doors or devices, with technical detail to enable staff to determine a remedy. For example, if a ligature-detection device of a door (such as the entrance door of Figure 2) has not responded to a recent status update request transmitted by computer apparatus 108, then this may identify a fault with said door. This fault can be recorded in fault list 306. Similarly, recent alarm events (which may be associated with a ligature-detection device being triggered) can be recorded and displayed in alarm list 308. Ward activity, including faults, alarms and potentially other data may also be consolidated and listed in a ward activity feed 310.

As described in further detail in relation to Figure 5 below, GUI 300 can be used to intuitively indicate where a new status change has occurred. Consider, for example, a trigger event at a ligature-detection device occurring at one of the monitored doors. This may be indicative that a person is attempting to secure a ligature to the door in question. Computer apparatus 108 may learn of this event either by receiving a direct message from the ligature-detection device (via the hardwired network) generated by the event or in response to polling the device or door in question with a status update request. In response to identifying that a status of the door has changed, computer apparatus 108 is configured to modify the manner in which that door is presented on the GUI 300.

In an example, the icon 304 provided at the door in question may begin to flash vigorously and/or change colour. A particular animation or colour change reserved for ligature alarms may be used to emphasise the severity of the situation. Computer apparatus 108 may also sound an auditory alarm at the display and/or at various alarms situated around the facility (such as via alarm system 112 of Figure 1). This alerts staff members to consult the nearest display showing GUI 300. By looking at the GUI, staff can immediately determine where the trigger event is occurring, because the icon 304 associated with the door in question has changed in appearance. In this way, staff are provided with an immediate and intuitive understanding of where in the building assistance is required. If different visual modifications are associated with different event types and status changes (e.g. door opening, fault, ligature trigger), then staff can also see immediately and at a glance determine the event type and severity of a status change. This can enable staff to triage and attend the most severe events first, in case more than one event happens simultaneously. Fault list 306 and alarm list 308 can provide further information relating to the new alert/alarm. In combination with the graphically displayed floor plan 302, this provides staff at-a-glance with all the information required to respond to an event quickly and effectively.

Additional information concerning the new alarm event is also recorded in ward activity feed 310. This can provide additional information to staff and can also enable complete and accurate recordal of data. For example, data from fault list 306, alarm list 308 and/or ward activity feed 310 can be recorded to a local or remote database by computer apparatus 108. This advantageously means that a single system (door monitoring system 100) is used to detect events, alert staff, and record event history logs. No interfacing between multiple third party systems is required, and event log data is available from the door monitoring system 100 directly. This avoids the need to interface with any third parties, thereby improving reliability and auditability.

As can be seen, therefore, GUI 300 and underlying door monitoring system 100 provide an intuitive and effective means of quickly alerting staff as to the nature and location of a door-related event or status change. The system also provides a reliable way of recording data for future analysis and auditing.

In the above-described examples, all ligature-detection events are treated equally. However, the present inventors have identified that in many instances it can be advantageous to differentiate between brief ligature-detection events and prolonged ones. By their nature, ligature-detection devices are often very sensitive, such that attempts to secure a ligature can be effectively identified. They may also be placed along more than one (often three) edges of a monitored door leaf. A drawback of this required sensitivity and extensive coverage, however, is that ligature-detection devices can frequently be triggered inadvertently, through benign actions. For example, a patient or staff member may brush against or bump into the edge of a door leaf when entering or exiting a room. Pressure may also be applied to the door edges during routine cleaning, for example, or through a variety of benign interactions with a door that are not associated with the attempt to attach a ligature. Each of these benign events will nevertheless typically trigger the ligature-detection device on the door in question.

The present inventors have identified that in many settings the disclosed system would be rendered unworkable if each of these benign events were to trigger a full alarm, for example one that is associated with an alarm bell or visual indicator. Many psychiatric wards or prisons do not have sufficient staff to attend to every such trigger event, given most are likely to be very short-lived and entirely benign. One response to this problem is to configure the system to simply ignore ligature-detection events that last less than a threshold duration. For example, in some cases trigger events lasting less than seven seconds are ignored. If the trigger event lasts for longer than seven seconds, then an alarm is sounded.

While this approach is effective at preventing false alarms, this approach also presents a problem in that it means that staff can be slow to respond to genuine, potentially life-threatening, ligature attachment events. In particular, because triggers below a threshold duration are ignored by the system, a patient or inmate may already have spent seven seconds (or however long the threshold is set to) attaching a ligature to their door before anyone is made aware of this happening. This presents a clear risk to safety.

The present inventors have therefore identified an alternative approach, which enables staff members to easily distinguish between brief, likely innocuous trigger events and potentially more serious events where a ligature-detection device is triggered for an extended period of time. In particular, the present inventors have identified that it can be beneficial to differentiate between so-called "pre-alarms" and "full alarms". A pre-alarm can be considered a brief ligature-detection event and a full alarm can be considered as a longer duration ligature-detection event. A threshold duration can be set which determines the transition from pre- to full- alarm.

As described above, the disclosed system may receive an indication from a monitored door that the ligature-detection device of the door has been triggered. In response to this initial trigger, a first type of alarm (a "pre-alarm") may be sounded. The pre-alarm may be relatively brief and relatively low-volume compared to a full alarm. The pre-alarm may therefore intuitively indicate a low priority event to a user (e.g. staff member) monitoring the facility. As well as, or instead of, the auditory indication, a pre-alarm may also result in a particular form of visual modification on a visual display. For example, the appearance of the door in question (or an icon associated therewith) may change to indicate that a pre-alarm event has been detected. In one example, the door may change colour to orange and may flash once.

The system can then monitor the door in question to determine how long the trigger-event lasts. In other words, the duration for which the ligature-detection device has been triggered can be determined. If the trigger event continues (i.e. the ligature-detection device continues to be activated or depressed) beyond a threshold duration, then the pre-alarm can transition to a full alarm. A full alarm may be represented by a relatively longer, relatively high-volume tone which is intuitively indicative of an urgent, high priority event. For example, in some examples a full alarm results in a loud, continuous or repeating auditory tone in contrast to a pre-alarm which results in a quiet, brief auditory tone.

Alternatively or additionally, a full-alarm may involve a particular form of visual modification on the visual display which allows it to be easily differentiated from pre-alarms. For example, the appearance of the door in question (or an icon associated therewith) may change to indicate that a full-alarm event has been detected. In one example, the door may change colour to red and/or may flash vigorously or continuously.

It will be appreciated that the disclosed functionality solves the above-described false alarm problem without compromising patient safety. In particular, the system enables brief, likely innocuous trigger events to be easily and intuitively distinguished from longer, potentially more serious trigger events. This functionality avoids the scenario where every slight trigger of the ligature-detection device results in a full-blown alarm being sounded. However, because pre-alarm events still result in some minor form of notification, staff are less likely to be taken unawares by a subsequent full alarm. As a result, an effective balance between avoiding false alarms and ensuring patient safety can be achieved.

The benefit of the disclosed approach may be further understood through consideration of the following illustrative examples. In a first scenario, a patient entering their room in a psychiatric ward touches the edge of their room door in an entirely benign manner. This is detected by the ligature-detection device on the door. As a result, a pre-alarm is sounded (via an audio indication and visual modification) which draws the attention of a supervisor who is monitoring the doors of the facility via a display. Because only a pre-alarm has been sounded, no response is yet required and resources are not wasted on investigating an innocuous detection event. In this example, no further triggers at the door in question are detected and so no further alarms are sounded. The supervisor can continue to monitor the facility as before.

In a second scenario, a patient in their room in a psychiatric ward begins attempting to attach a ligature to their door. This will generally require touching the edges of the door several times as the person attempts to attach the ligature securely. Each touch, even if short, will result in a pre-alarm. While one pre-alarm may not be suspicious, several pre-alarms in short succession are. As a result, the supervisor of the facility is forewarned of a potential ligature attachment event. Supervisors can similarly be forewarned if a patient is tampering with the ligature-detection system, which will similarly be characterised by several detection events in quick succession. This pre-alarm functionality thus enables pre-emptive investigation of the room in question. If the succession of pre-alarms is then followed shortly after by a full alarm, the supervisor has good reason to believe that the event that has been detected is indeed a real ligature-attachment event. Because a supervisor or staff member has been alerted to this in a systematic and intuitive manner, they are able to administer assistance more rapidly than if no pre-alarm had been sounded prior to the full alarm.

As can be seen, the disclosed functionality enables reduced false alarms, enables innocuous touch events to be distinguished easily and intuitively from more suspicious events, and enables supervisors and staff members to be forewarned of potential ligature attachment events in a way that would not be possible if short-duration events were simply suppressed or muted.

Even if pre-alarms are not associated with any auditory or visual indication, the differentiation between pre- and full alarms can still be useful. For example, the system may monitor how many pre-alarm events occur at a given door in a particular time-frame. If more than a threshold number of pre-alarm events occur in the time-frame, then this may be deemed suspicious behaviour and the system can notify a user (e.g. supervisor) via a display, for example in a notification or by drawing the user's attention to the door in question via a visual modification or alert.

The present inventors have identified that the threshold duration for delimitating between pre- and full- alarms should advantageously be 2 seconds or more. Most benign triggerings of the ligature-detection device last less than 2 seconds, and so having a threshold duration of 2 seconds or more ensures that false alarms are effectively avoided.

The present inventors have also identified that the threshold duration for delimitating between pre and full alarms should advantageously be 7 seconds or less. If the threshold is higher than 7 seconds, then the system may be too slow to trigger a full alarm in response to an actual ligature attachment event where a person is attempting to secure a ligature to the door in question.

Hence, advantageously the threshold duration which defines the boundary between a pre- and full- alarm is between 2 and 7 seconds inclusive. The present inventors have identified that a threshold of 7 seconds is particularly suitable, ensuring an effective balance between avoiding false alarms and ensuring patient or inmate safety.

Turning now to Figure 4, a method of configuring door monitoring system 100 is shown. The method may be performed at a computer apparatus such as computer apparatus 108.

At block 402, the method involves displaying a floor plan of a building comprising one or more doors each comprising a ligature-detection device. The floor plan may be of the type shown in Figure 3 and may be provided as part of GUI 300. The floor plan may be displayed on a computer screen of computer apparatus 108 or at a remote display such as a television (as shown in the example of Figure 2), which may be located at a particular location in the building where staff members are likely to see it regularly.

At block 404, the method involves receiving a user input indicative of a selection of one of the doors comprising a ligature-detection device. In an example, selection of a door involves a user using drag-and-drop functionality to drag an icon (such as one of icons 304 in Figure 3) onto a door. This designates the selected door for monitoring. Identification of the selected door may be facilitated through a software application running on computing apparatus 108 (as shown in Figure 2) which is configured to associate the location of a user input on GUI 300 with the output of a particular door.

For example, when a location on the GUI is selected, for example when an icon 304 is dropped at that location, the computer apparatus 108 is thereby able to associate the selected door with a monitoring protocol. In an example, this involves associating an output of the selected door (such as outputs of sensors associated with the door) with an IP address for receiving status updates from the selected door. This enables computing apparatus 108 to receive status updates from the selected door, thereby enabling the door to be monitored at block 406. As discussed above, a hardwired connection may be established between the selected door and computing apparatus 108 at block 406 to enable said monitoring in a more secure and reliable manner.

In an example, the user input also includes an indication of which status of the selected door is to be monitored. In other words, the user input may indicate whether the system should monitor specific door sensors 102, the door's ligature-detection device or all available sensors. This determination is used by the system to determine which outputs from the selected door (or, more accurately, from the sensors and/or ligature-detection device of the door) are to be obtained and monitored. For example, if the user input indicates that all sensors are to be monitored, then computing apparatus 108 will obtain any and all sensor-related data available from the selected door. If, on the other hand, the user input indicates that only ligature-detection devices are to be monitored, then computing apparatus 108 will obtain only data available from the selected door relating to ligature detection. In that case, other data such as relating to door opening events will be ignored or not fetched. The statuses which are to be monitored may be indicated by the type of icon 304 which is dragged and dropped onto the GUI 300 by the user.

In the example shown in Figure 4, monitoring of the door comprises determining, at block 408, whether a status of the selected door has changed. This may be achieved by sending regular status update requests to the door. Based on the door's response, its status can be determined by computer apparatus 108. If the door status has not changed since the previous update, monitoring continues. However, in response to determining that a status of the selected door has changed, the manner in which the selected door is presented on the displayed floor plan 302 of GUI 300 is modified. As described above, this modification may involve visually emphasising the door, changing a colour associated with the door and/or adding the door to an appropriate displayed list such as fault list 306 or alarm list 308. It will be apparent that modifying the manner in which the selected door is presented on the displayed floor plan may involve modifying the appearance of an icon associated with that door (such as icon 304) as opposed to, or in addition to, modifying the appearance of the door on the GUI itself. In addition to a visual modification, an auditory indication of the change of status of the door may also be provided.

As described above, the statuses of the door that can be monitored at block 406 can include: whether the selected door is open or closed; whether the ligature-detection device of the selected door has been triggered; whether the status of the selected door or of the or ligature-detection device can be accurately determined; and whether the selected door has responded to the status update request.

Turning now to Figure 5, a method of identifying a change of status at a monitored door is shown. The method may be performed at a computer apparatus such as computer apparatus 108, typically the same computer apparatus 108 as performed the method of Figure 4. Figure 5 may therefore be considered an extension of Figure 4, in particular to blocks 408 and 410. The method of Figure 4 may be considered a 'setup/configuration phase' while the method of Figure 5 may be considered a 'use phase' of the door monitoring system 100.

The method of Figure 5 begins, at block 502, by displaying a floor plan of a building comprising one or more monitored doors, each monitored door comprising a ligature-detection device. The monitored doors may be those selected in the method of Figure 4 at block 404. The monitored doors may be displayed on a floor plan similar to floor plan 302 of GUI 300 shown in Figure 3. In an example, the monitored doors are visually marked as being monitored by icons such as icons 304 in Figure 3. These icons may have been dragged-and-dropped onto the doors to designate the doors for monitoring during the method of Figure 4.

The method of Figure 5 continues by identifying, at block 504, that a status of a first monitored door of the one or more monitored doors has changed. This is analogous to the "yes" arm of block 408 of Figure 4. As described above, identifying that a status a door has changed can comprise identifying a variety of events or status changes at the door. The identification can result from transmittal of periodic status update requests from computing apparatus 108 to all monitored doors.

In one example, the status change indicates that the monitored door has been opened or closed. This may occur innocuously when a staff member enters or exits a room, however such an event may also be indicative of a patient/inmate attempting to escape or barricading themselves in a room they are not permitted to be in.

In another example, the status change indicates that that the ligature-detection device of the monitored door has been triggered. This may be indicative of a person attempting to secure a ligature to a door frame to cause self-harm.

In another example, the status change indicates that a fault with the ligature-detection device of the monitored door has been detected. This may occur due to wear-and-tear of the ligature-detection device or due to tampering.

In another example, the status change indicates the state of the monitored door or of its ligature-detection device can no longer be accurately determined or that the ligature-detection device (or another sensor or component of the monitored door) has not responded to a status update request. This may occur if there has been a signal failure, power outage or due to tampering.

Whatever the cause of the status change, staff will generally need to investigate and remedy the problem. Accordingly, the door monitoring system should alert them accordingly via the GUI 300. This is achieved by indicating, at block 506, that a status of the monitored door has changed. This is achieved by modifying the manner in which the monitored door is presented on the displayed floor plan. The same functionality may be utilised at block 506 as was discussed above in relation to block 410. In particular, either the door or an icon (such as icon 304) associated with the door on GUI 300 is modified to accentuate or visually emphasise the door, in order to draw staff members' attention to that location. An auditory alarm may also be sounded.

As noted above, the visual modification and/or auditory sound may differ based on which aspect of the monitored door's status has changed. In particular, the modification may be based on the severity or risk-level of the detected status change. Risk levels associated with certain status changes can be configured or pre-programmed during setup or configuration of the door monitoring system. For example, opening or closing of a door may be deemed a low-risk event. Hence, when such a status change is identified, an icon 304 associated with the opened door may flash green and no auditory alarm may be sounded. A fault with a ligature-detection device or other door sensor (or non-response therefrom) may be considered a medium-risk event. Hence, an icon 304 associated with a door having such a fault may flash yellow and a brief auditory alarm may be sounded. Triggering of a ligature-detection device may be considered a high-risk event. Hence, an icon 304 associated with a door having such a fault may flash red and expand, and a persistent auditory alarm may be sounded. It will be appreciated that these are merely examples of how visual and auditory cues can be used to intuitively communicate a door status change to staff. Many modifications and variations of this scheme will be apparent to a skilled reader and can be implemented within the disclosed systems and methods.

Optionally, the method may further comprise logging, at block 508, an indication that a status of the monitored door has changed. This may be recorded by computer apparatus 108 in a local or remote database to facilitate auditing of event histories.

As can be seen, the disclosed methods and systems provide a more intuitive and reliable mechanism for both configuring and using a door monitoring system in an environment containing doors having ligature-detection devices. In particular, by placing the doors in context in a displayed floor plan, the layout and location of the doors is more easily understood. Staff can thereby more easily and effectively configure the door monitoring system and respond to status changes at specific doors more quickly and appropriately.

As well as monitoring door status changes, the disclosed systems and methods can also be used to monitor other information that is important for ensuring staff and patient/inmate safety and for monitoring and auditing quality of care. The data generated by any of these additional monitoring processes can be incorporated into the systems described previously and can be displayed and recorded in any suitable manner as described above with reference to door status changes. Hence, all of the functionality described above with reference to how door status changes can be detected, visualised and processed apply also to data generated by other monitoring activities such as those described below.

By monitoring various types of information in this way and recording all data though a single streamlined system, the disclosed systems and methods can provide contextually rich information for a variety of events that can occur in a psychiatric ward, prison or other similar environment. Staff can access all of this data through a single system, improving usability and avoiding confusion. The disclosed implementations can also provide a single record or database for auditing purposes which means there is no need to audit or interface with several different systems. Some example implementations of such functionality for monitoring other aspects besides door status changes will now be briefly described.

A first implementation relates to recording staff attack alarm data and/or patient call data. The above systems and methods for monitoring status changes at doors can be similarly used to monitor, display and record such staff attack alarm data and/or patient call data.

Turning first to staff attack alarm data, staff working in psychiatric wards and prisons often carry staff alarm devices which may comprise fobs or devices comprising a button or trigger which can be actuated in case of an attack by a patient or inmate. The staff alarm devices may be connected or monitored via geo-tracking technology such that the location of each particular staff alarm device can be determined. Unfortunately, existing systems for alerting other staff or security personnel of such staff alarm events are typically rudimentary at best. One example of an existing staff attack alarm system comprises alarm display panels. In response to a staff attack alarm, these display panels provide a textual readout of where an alarm event has been triggered, such as "staff alarm room 10". However, as described above in relation to door alarms, such rudimentary textual alerts do not provide staff with adequate information to respond quickly. In particular, staff may not know where "room 10" is located, may not know the quickest way to get there or may not be sure of their precise current location relative to room 10.

The systems and methods of the present disclosure allow for an improved interface, whereby staff attack alarm events (i.e. status changes detected at staff alarm devices) are displayed on the graphical display (such as the GUI shown in Figure 3) in a similar manner as the door status changes described above. In particular, responsive to identifying a staff attack alarm (i.e. an actuation of a monitored staff alarm device), such an event may be visually indicated in the appropriate location on the displayed floor plan of the present disclosure to show where the event is happening. In other words, the location of the device as displayed on the displayed floor plan corresponds to the determined location of the device which triggered the alarm or recorded a change in status. This provides geographical and spatial context for the event, enabling other staff and security personnel to respond more quickly and effectively, thereby improving staff safety. Staff attack alarms may be presented on the displayed floor plan in any suitable manner, for example a vigorously flashing icon may appear on the display at the appropriate location on the floor plan. The visual alert may be accompanied by an urgent (e.g. loud and/or prolonged) auditory sound. Preferably, staff attack alarms events such as these are presented on the display in a different manner (e.g. different colour, shape, symbol or intensity) than door status changes, such that staff can easily and intuitively tell the difference between types of event. All of the above-described functionality described in the context of door status changes (e.g. a pre-alarm threshold and/or a recording alarm events in a list for future auditing) can be used also in the context of staff attack alarms. If staff alarm devices are portable, their location can be monitored using any suitable tracking technology such as Bluetooth^{®}, GPS or other similar technology to enable real-time or periodic location tracking. Alternatively, hardwired staff alarm devices may be provided at fixed locations that are known to the door monitoring system (e.g. following a configuration phase, similar to that described above in relation to configuring the system to monitoring doors). For hardwired devices, end-of-line resistors can be provided (as described above for door alarms) to enable faults or errors with device to be identified and alerted to the door monitoring system.

In addition/alternatively to recording and displaying staff attack alarm events, the system and method of the present disclosure can be used to detect, display and record patient/inmate call events. For example, a patient in a bedroom may depress a button on a patient call device to call for assistance. Such an event can be recorded and shown, in the context of the floor plan, on the display. In other words, the location of the device as displayed on the displayed floor plan again corresponds to the determined location of the device which triggered the alarm or recorded a change in status. Once again, patient/inmate call alarms can be distinguished in terms of their audio-visual representation on the display from other types of alarm such as staff attack events or door status changes. In some examples, patient call alarms may generate a slow pulsing visual indicator and a low intensity (e.g. quiet or short) auditory indication. Again, the location of patient call devices can be monitored (if portable) or can be programmed into the system at configuration time if the device is placed at a predetermined position in each patient's room (e.g. on the wall). For hardwired devices, end-of-line resistors can be provided (as described above for door alarms) to enable faults or errors with device to be identified and alerted to the door monitoring system.

A further aspect of functionality which can be incorporated into the systems and methods of the present disclosure relates to monitoring door observation panels provided in patient/inmate doors. In prisons or psychiatric wards, patient/inmate rooms will often contain such door observation panels (e.g. viewing windows) through which staff can monitor the occupant of the room for safety, security and wellbeing reasons. For reasons relating to privacy and occupant comfort, the viewing window is typically closed for the majority of the time and only opened when staff perform a check on the occupant. It is important therefore to monitor how often the viewing window is being opened by staff to ensure that patients/inmates are being checked in on frequently. This ensures quality and consistency of care.

The presently disclosed systems and methods can be used to monitor and record such viewing events at each door observation panel. In particular, the system can be configured to monitor door observation panels in a similar manner to how the system can monitor door sensors and/or patient and staff call devices. Observation panels can be opened in a number of ways, for example manually using a thumb-turn or handle or electronically using a button. Each such actuation can be recorded by the door monitoring system and stored alongside suitable date, time and location data for future auditing. If desired, such events can also be displayed on the displayed floor plan. This provides an electronic, reliable record of how often each viewing window has been opened, preventing staff from fraudulently recording that patient/inmate observations have taken place when they have not. This ensures patient/inmate safety and quality of care. As above, end-of-line resistors can be provided to enable faults or errors with the door observation panels to be identified and alerted to the door monitoring system.

A further aspect of functionality which can be incorporated into the systems and methods of the present disclosure relates to monitoring wearables worn by staff and/or occupants. Such wearables can be in the form of existing wearable technology (e.g. a smartwatch) or a bespoke wearable (e.g. wrist band) used by the institution where the monitoring system is provided. Such wearables can enable geo-location tracking (e.g. using Bluetooth^{®}, GPS or other similar technology to enable real-time or periodic location tracking) for tracking the location of staff and/or patients/inmates within the facility. The locations of persons can, if desired, be displayed on the floor plan of the door monitoring system to provide security personnel with up-to-date information on staff or occupant whereabouts. For example, if staff want to locate a particular patient or inmate, the system can be used to ping their wearable and the last known location of the wearable can be displayed on the display in the context of the floor plan.

Wearables can also be used to monitor vital signs (e.g. heartrate) for monitoring purposes. This is particularly useful for monitoring the health and wellbeing of, for example, inmates at a psychiatric ward. It is important to monitor the vital signs of such patients frequently, however it may be undesirable to require staff to manually check the patients multiple times per day. A wearable provides a less intrusive mechanism for monitoring the wellbeing of the occupant. In some cases, an unexpected or suspicious change in occupant vital signs can also be identified and flagged to staff using the systems (e.g. displayed floor plan) of the present disclosure. For example, as before the location of the device as displayed on the displayed floor plan again corresponds to the determined location of the device which triggered the alarm or recorded a change in status. For example, a raised heart rate in a patient may indicate distress or even that the patient is about to attempt self-harm. By flagging such events using the disclosed systems, staff can be alerted and respond quickly. For example, if a raised heartrate is detected then a suitable visual indicator may appear at the location of that person on the display of the door monitoring system (e.g. the GUI of Figure 3) to draw staff attention and immediately show where the distressed person is located. Patient vital signs can also be recorded for future auditing purposes.

An alternative to wearables which provides similar benefits and can similarly be monitored using the door monitoring system include other vital sign monitors such as bedsheets incorporating sensors (e.g. vibration sensors). Such devices enable occupants to be monitored at all times, including at night, without disturbing them. Such devices also provide a more suitable and less intrusive alternative for monitoring room occupants than cameras, which are typically not suitable for providing in occupant bedrooms due to privacy reasons. The data from any of these devices can be incorporated into the systems described previously and can be displayed and recorded in any suitable manner as described above with reference to door status changes.

Turning finally to Figure 6, Figure 6 shows a schematic and simplified representation of a computer apparatus 600 which can be used to perform the methods described herein, either alone, in combination with other computer apparatuses or as part of a "cloud" computing arrangement. In particular, computer apparatus 600 may be the same as, or provide the functionality discussed in relation to, computer apparatus 108 discussed above with reference to Figures 1-5.

The computer apparatus 600 comprises various data processing resources such as a processor 602 (in particular a hardware processor) coupled to a central bus structure. Also connected to the bus structure are further data processing resources such as memory 604. A display adapter 606 connects a display device 608 to the bus structure. One or more user-input device adapters 610 connect a user-input device 612, such as a keyboard and/or a mouse to the bus structure. One or more communications adapters 614 are also connected to the bus structure to provide connections to other computer systems 600 and other networks, in particular the other components and network devices of the door monitoring system 100.

In operation, the processor 602 of computer apparatus 600 executes a computer program comprising computer-executable instructions that may be stored in memory 604. When executed, the computer-executable instructions may cause the computer system 600 to perform one or more of the methods described herein, in particular those described in relation to Figures 4 and 5 above. The results of the processing performed may be displayed to a user via the display adapter 606 and display device 608. In particular, this may involve display of GUI 300 described in relation to Figure 3. User inputs for controlling the operation of the computer apparatus 600, for example during the setup phase described in relation to Figure 4, may be received via the user-input device adapters 610 from the user-input devices 612.

It will be apparent that some features of computer apparatus 600 shown in Figure 6 may be absent in certain cases. For example, one or more of the plurality of computer apparatuses 600 may have no need for display adapter 606 or display device 608. This may be the case, for example, for particular server-side computer apparatuses 600 which are used only for their processing capabilities and do not need to display information to users. For example, computers configured to record event logs recorded by door monitoring system 100 may be of this nature. Similarly, user input device adapter 610 and user input device 612 may not be required in some cases. In its simplest form, computer apparatus 600 comprises processor 602 and memory 604.

The above detailed description describes a variety of exemplary arrangements of and methods of implementing and using a door monitoring system. However, the described arrangements and methods are merely exemplary, and it will be appreciated by a person skilled in the art that various modifications can be made without departing from the scope of the appended claims. Some of these modifications will now be briefly described, however this list of modifications is not to be considered as exhaustive, and other modifications will be apparent to a person skilled in the art.

Whilst the examples of psychiatric hospitals and prisons are referred to throughout, these are merely examples. The presently disclosed systems and methods will find utility in any setting housing persons at risk of self-harm where doors having ligature-detection devices are provided.

It will be appreciated that door sensors 102 described above can comprise additional types of sensor to those described. Door sensors 102 may be more generally considered as, or may comprise, alarm trigger interfaces, configured to determine if an alarm or fault state has occurred at a door.

It will be appreciated that the networks shown in Figures 1 and 2 are schematic and simplified. In reality, there may be numerous versions of the components shown as well as additional components. Some of the components, in particular one or more computer apparatus 108, may be provided off-site and can interface with the remaining components through network switch 106 or via a similar mechanism.

The GUI showcased in Figure 3 is merely one example of how the presently disclosed methods can be implemented. It will be appreciated that any suitable floor plan design can be used. Similarly, any suitable icon or visual indicator for showing that a particular door is being monitored can be used, and these can be added to the GUI by the user in any suitable manner not limited to drag-and-drop. The position, style and size of all GUI elements shown is merely exemplary, as are the visual modifications discussed above which can be modified as required by each use case.

While various specific combinations of components and method steps have been described, these are merely examples. Components and method steps may be combined in any suitable arrangement or combination. Components and method steps may also be omitted to leave any suitable combination of components or method steps.

The described methods may be implemented using computer executable instructions. A computer program product or computer readable medium may comprise or store the computer executable instructions. The computer program product or computer readable medium may comprise a hard disk drive, a flash memory, a read-only memory (ROM), a CD, a DVD, a cache, a random-access memory (RAM) and/or any other storage media in which information is stored for any duration (e.g., for extended time periods, permanently, brief instances, for temporarily buffering, and/or for caching of the information). A computer program may comprise the computer executable instructions. The computer readable medium may be a tangible or non-transitory computer readable medium. The term "computer readable" encompasses "machine readable".

The singular terms "a" and "an" should not be taken to mean "one and only one". Rather, they should be taken to mean "at least one" or "one or more" unless stated otherwise. The word "comprising" and its derivatives including "comprises" and "comprise" include each of the stated features, but does not exclude the inclusion of one or more further features.

The above implementations have been described by way of example only, and the described implementations are to be considered in all respects only as illustrative and not restrictive. It will be appreciated that variations of the described implementations may be made without departing from the scope of the disclosure. It will also be apparent that there are many variations that have not been described, but that fall within the scope of the appended claims.

## Claims

1. A computer-implemented method of identifying, by a door monitoring system (100), a change of status at a monitored door, the method comprising:
displaying (502) a floor plan (302) of a building comprising one or more monitored doors, each monitored door comprising a ligature-detection device, wherein each monitored door is monitored via a hardwired connection between the respective door and a computing apparatus of the door monitoring system whereby data from each monitored door may be obtained via an end-to-end, hardwired connection;
identifying (504) that a status of a first monitored door of the one or more monitored doors has changed, wherein identifying that a status of the first monitored door has changed comprises determining that:
a) the ligature-detection device of the first monitored door has been triggered;
b) a fault with the ligature-detection device of the first monitored door has been detected;
c) the state of the ligature-detection device of the first monitored door can no longer be accurately determined; and/or
d) the ligature-detection device of the first monitored door has not responded to a status update request, and
indicating (506) that a status of the first monitored door has changed by modifying the manner in which the first monitored door is presented on the displayed floor plan.

2. The method of claim 1, the method further comprising:
responsive to identifying that a status of the first monitored door has changed, causing an auditory indication to be sounded.

3. The method of claim 1 or 2, the method further comprising:
responsive to determining that the ligature-detection device of the first monitored door has been triggered, determining a duration for which the ligature-detection device has been triggered.

4. The method of claim 3 when dependent on claim 2, wherein the auditory indication is based on the duration for which the ligature-detection device has been triggered.

5. The method of claim 4, wherein:
when the ligature-detection device has been triggered for less than a threshold duration, a first type of auditory indication is sounded; and
when the ligature-detection device has been triggered for more than a threshold duration, a second type of auditory indication is sounded.

6. The method of any of claims 2-5, wherein the manner in which the first monitored door is presented on the displayed floor plan is modified based on the duration for which the ligature-detection device has been triggered.

7. The method of claim 6, wherein the manner in which the first monitored door is presented on the displayed floor plan is modified such that:
when the ligature-detection device has been triggered for less than a threshold duration, a first type of visual modification is applied; and
when the ligature-detection device has been triggered for more than a threshold duration, a second type of visual modification is applied.

8. The method of any preceding claim, wherein modifying the manner in which the first monitored door is presented on the displayed floor plan comprises at least one of:
visually emphasising the first monitored door;
changing a colour associated with the first monitored door; and
adding the first monitored door to a displayed list of doors associated with having an alarm or having a fault.

9. The method of any preceding claim further comprising:
logging, in a database, an indication that a status of the first monitored door has changed.

10. The method of any preceding claim, further comprising periodically polling the one or more monitored doors for an indication of their respective statuses.

11. The method of any preceding claim, further comprising:
identifying that a status of a monitored staff alarm device or patient call device has changed; and
indicating that a status of the monitored staff alarm device or patient call device has changed by modifying the manner in which the staff alarm device or patient call device is presented on the displayed floor plan.

12. The method of any preceding claim, further comprising:
identifying that a monitored door observation panel has been opened; and
recording in memory an indication that the monitored door observation panel has been opened.

13. The method of any preceding claim, further comprising:
identifying that a status of a monitored vital sign monitoring device has changed; and
indicating that a status of the monitored vital sign monitoring device has changed by modifying the manner in which the vital sign monitoring device is presented on the displayed floor plan.

14. A computer-implemented method of configuring a door monitoring system (100), the method comprising:
displaying (402) a floor plan (302) of a building comprising one or more doors each comprising a ligature-detection device;
receiving (404) a user input indicative of a selection of one of the one or more doors; and
based on the user input, monitoring (406) a status of the selected door, wherein monitoring a status of the selected door comprises monitoring whether the ligature-detection device of the selected door has been triggered, and
wherein, based on the user input, a hardwired connection is established between the selected door and a computing apparatus of the door monitoring system whereby data from the selected door may be obtained via an end-to-end, hardwired connection.

15. A computer apparatus configured to perform the method of any preceding claim.

16. A computer-readable medium comprising instructions which, when executed by a computer, cause the computer to carry out the method of any of claims 1-14.

17. A computer program comprising instructions which, when the program is executed by a computer, cause the computer to carry out the method of any of claims 1-14.

## Patentansprüche

1. Computerimplementiertes Verfahren zum Identifizieren, durch ein Türüberwachungssystem (100), einer Änderung eines Status bei einer überwachten Tür, wobei das Verfahren umfasst:
Anzeigen (502) eines Grundrisses (302) eines Gebäudes, das eine oder mehrere überwachte Türen umfasst, wobei jede überwachte Tür eine Ligaturdetektionsvorrichtung umfasst, wobei jede überwachte Tür über eine festverdrahtete Verbindung zwischen der jeweiligen Tür und einer Recheneinrichtung des Türüberwachungssystems überwacht wird, wobei Daten von jeder überwachten Tür über eine festverdrahtete Ende-zu-Ende-Verbindung erhalten werden können;
Identifizieren (504), dass sich ein Status einer ersten überwachten Tür der einen oder der mehreren überwachten Türen geändert hat, wobei das Identifizieren, dass sich ein Status der ersten überwachten Tür geändert hat, das Bestimmen umfasst, dass:
a) die Ligaturdetektionsvorrichtung der ersten überwachten Tür ausgelöst wurde;
b) ein Fehler an der Ligaturdetektionsvorrichtung der ersten überwachten Tür detektiert wurde;
c) der Zustand der Ligaturdetektionsvorrichtung der ersten überwachten Tür nicht mehr genau bestimmt werden kann; und/oder
d) die Ligaturdetektionsvorrichtung der ersten überwachten Tür nicht auf eine Statusaktualisierungsanforderung geantwortet hat, und
Angeben (506), dass sich ein Status der ersten überwachten Tür geändert hat, durch Modifizieren der Art und Weise, mit der die erste überwachte Tür auf dem angezeigten Grundriss dargestellt wird.

2. Verfahren nach Anspruch 1, wobei das Verfahren ferner umfasst:
als Reaktion auf das Identifizieren, dass sich ein Status der ersten überwachten Tür geändert hat, Bewirken, dass eine akustische Angabe ertönt wird.

3. Verfahren nach Anspruch 1 oder 2, wobei das Verfahren ferner umfasst:
als Reaktion auf das Bestimmen, dass die Ligaturdetektionsvorrichtung der ersten überwachten Tür ausgelöst wurde, Bestimmen einer Dauer, für die die Ligaturdetektionsvorrichtung ausgelöst wurde.

4. Verfahren nach Anspruch 3, wenn abhängig von Anspruch 2, wobei die akustische Angabe auf der Dauer basiert, für die die Ligaturdetektionsvorrichtung ausgelöst wurde.

5. Verfahren nach Anspruch 4, wobei:
wenn die Ligaturdetektionsvorrichtung für weniger als eine Schwellendauer ausgelöst wurde, eine erste Art von akustischer Angabe ertönt wird; und
wenn die Ligaturdetektionsvorrichtung für mehr als eine Schwellendauer ausgelöst wurde, eine zweite Art von akustischer Angabe ertönt wird.

6. Verfahren nach einem der Ansprüche 2-5, wobei die Art und Weise, mit der die erste überwachte Tür auf dem angezeigten Grundriss dargestellt wird, basierend auf der Dauer modifiziert wird, für die die Ligaturdetektionsvorrichtung ausgelöst wurde.

7. Verfahren nach Anspruch 6, wobei die Art und Weise, mit der die erste überwachte Tür auf dem angezeigten Grundriss dargestellt wird, so modifiziert wird, dass:
wenn die Ligaturdetektionsvorrichtung für weniger als eine Schwellendauer ausgelöst wurde, eine erste Art von visueller Modifikation angewendet wird; und
wenn die Ligaturdetektionsvorrichtung für mehr als eine Schwellendauer ausgelöst wurde, eine zweite Art von visueller Modifikation angewendet wird.

8. Verfahren nach einem der vorhergehenden Ansprüche, wobei das Modifizieren der Art und Weise, mit der die erste überwachte Tür auf dem angezeigten Grundriss dargestellt wird, mindestens eines von Folgendem umfasst:
visuelles Hervorheben der ersten überwachten Tür;
Ändern einer Farbe, die mit der ersten überwachten Tür assoziiert ist; und
Hinzufügen der ersten überwachten Tür zu einer angezeigten Liste von Türen, die mit einem Alarm oder einem Fehler assoziiert sind.

9. Verfahren nach einem der vorhergehenden Ansprüche, umfassend:
Protokollieren, in einer Datenbank, einer Angabe, dass sich ein Status der ersten überwachten Tür geändert hat.

10. Verfahren nach einem der vorhergehenden Ansprüche, ferner umfassend periodisches Abfragen der einen oder der mehreren überwachten Türen nach einer Angabe ihrer jeweiligen Status.

11. Verfahren nach einem der vorhergehenden Ansprüche, ferner umfassend:
Identifizieren, dass sich ein Status einer überwachten Personalalarmvorrichtung oder Patientenrufvorrichtung geändert hat; und
Angeben, dass sich ein Status der überwachten Personalalarmvorrichtung oder Patientenrufvorrichtung geändert hat, durch Modifizieren der Art und Weise, mit der die Personalalarmvorrichtung oder Patientenrufvorrichtung auf dem angezeigten Grundriss dargestellt wird.

12. Verfahren nach einem der vorhergehenden Ansprüche, ferner umfassend:
Identifizieren, dass eine überwachte Türbeobachtungsplatte geöffnet wurde; und
Aufzeichnen, in einem Speicher, einer Angabe, dass die überwachte Türbeobachtungsplatte geöffnet wurde.

13. Verfahren nach einem der vorhergehenden Ansprüche, ferner umfassend:
Identifizieren, dass sich ein Status einer überwachten Vitalzeichenmonitorvorrichtung geändert hat; und
Angeben, dass sich ein Status der überwachten Vitalzeichenmonitorvorrichtung geändert hat, durch Modifizieren der Art und Weise, mit der die Vitalzeichenmonitorvorrichtung auf dem angezeigten Grundriss dargestellt wird.

14. Computerimplementiertes Verfahren zum Konfigurieren eines Türüberwachungssystems (100), wobei das Verfahren umfasst:
Anzeigen (402) eines Grundrisses (302) eines Gebäudes, das eine oder mehrere Türen umfasst, die jeweils eine Ligaturdetektionsvorrichtung umfassen;
Empfangen (404) einer Benutzereingabe, die eine Auswahl von einer der einen oder der mehreren Türen angibt; und
basierend auf der Benutzereingabe, Überwachen (406) eines Status der ausgewählten Tür, wobei das Überwachen eines Status der ausgewählten Tür das Überwachen umfasst, ob die Ligaturdetektionsvorrichtung der ausgewählten Tür ausgelöst wurde, und
wobei, basierend auf der Benutzereingabe, eine festverdrahtete Verbindung zwischen der ausgewählten Tür und einer Recheneinrichtung des Türüberwachungssystems hergestellt wird, wobei Daten von der ausgewählten Tür über eine festverdrahtete Ende-zu-Ende-Verbindung erhalten werden können.

15. Computereinrichtung, die dazu ausgelegt ist, das Verfahren nach einem der vorhergehenden Ansprüche durchzuführen.

16. Computerlesbares Medium, das Anweisungen umfasst, die bei Ausführung durch einen Computer den Computer veranlassen, das Verfahren nach einem der Ansprüche 1-14 auszuführen.

17. Computerprogramm, das Anweisungen umfasst, die, wenn das Programm durch einen Computer ausgeführt wird, den Computer veranlasst, das Verfahren nach einem der Ansprüche 1-14 auszuführen.

## Revendications

1. Procédé mis en œuvre par ordinateur pour identifier, au moyen d'un système de surveillance de portes (100), un changement d'état au niveau d'une porte surveillée, le procédé comprenant :
l'affichage (502) d'un plan d'étage (302) d'un bâtiment comprenant une ou plusieurs portes surveillées, chaque porte surveillée comprenant une dispositif de détection de ligature, chaque porte surveillée étant surveillée par l'intermédiaire d'une connexion câblée entre la porte respective et un appareil informatique du système de surveillance de portes, selon lequel des données provenant de chaque porte surveillée peuvent être obtenues par l'intermédiaire d'une connexion câblée de bout à bout ;
l'identification (504) qu'un état d'une première porte surveillée des une ou plusieurs portes surveillées a changé, l'identification qu'un état de la première porte surveillée a changé comprenant la détermination que :
a) le dispositif de détection de ligature de la première porte surveillée a été déclenché ;
b) une défaillance du dispositif de détection de ligature de la première porte surveillée a été détectée ;
c) l'état du dispositif de détection de ligature de la première porte surveillée ne peut plus être déterminé avec précision ; et/ou
d) le dispositif de détection de ligature de la première porte surveillée n'a pas répondu à une demande de mise à jour d'état, et
l'indication (506) qu'un état de la première porte surveillée a changé en modifiant la manière dont la première porte surveillée est présentée sur le plan d'étage affiché.

2. Procédé selon la revendication 1, le procédé comprenant en outre :
en réponse à l'identification qu'un état de la première porte surveillée a changé, l'entraînement du retentissement d'une indication auditive.

3. Procédé selon la revendication 1 ou 2, le procédé comprenant en outre :
en réponse à la détermination que le dispositif de détection de ligature de la première porte surveillée a été déclenché, la détermination d'une durée pendant laquelle le dispositif de détection de ligature a été déclenché.

4. Procédé selon la revendication 3 lorsqu'elle dépend de la revendication 2, dans lequel l'indication auditive est basée sur la durée pendant laquelle le dispositif de détection de ligature a été déclenché.

5. Procédé selon la revendication 4, dans lequel :
lorsque le dispositif de détection de ligature a été déclenché pendant une durée inférieure à une durée seuil, un premier type d'indication auditive est émis ; et
lorsque le dispositif de détection de ligature a été déclenché pendant une durée supérieure à une durée seuil, un deuxième type d'indication auditive est émis.

6. Procédé selon l'une quelconque des revendications 2 à 5, dans lequel la manière dont la première porte surveillée est présentée sur le plan d'étage affiché est modifiée en fonction de la durée pendant laquelle le dispositif de détection de ligature a été déclenché.

7. Procédé selon la revendication 6, dans lequel la manière dont la première porte surveillée est présentée sur le plan d'étage affiché est modifiée de telle sorte que :
lorsque le dispositif de détection de ligature a été déclenché pendant une durée inférieure à une durée seuil, un premier type de modification visuelle est appliqué ;
lorsque le dispositif de détection de ligature a été déclenché pendant une durée supérieure à une durée seuil, un deuxième type de modification visuelle est appliqué.

8. Procédé selon l'une quelconque des revendications précédentes, dans lequel la modification de la manière dont la première porte surveillée est présentée sur le plan d'étage affiché comprend au moins un parmi :
l'accentuation visuelle de la première porte surveillée ;
le changement d'une couleur associée à la première porte surveillée ; et
l'ajout de la première porte surveillée à une liste affichée des portes associées à une alarme ou à un défaut.

9. Procédé selon l'une quelconque des revendications précédentes, comprenant en outre :
l'enregistrement, dans une base de données, d'une indication qu'un état de la première porte surveillée a changé.

10. Procédé selon l'une quelconque des revendications précédentes, comprenant en outre l'interrogation périodique des une ou plusieurs portes surveillées sur une indication de leurs états respectifs.

11. Procédé selon l'une quelconque des revendications précédentes, comprenant en outre :
l'identification qu'un état d'un dispositif d'alarme de personnel surveillé ou un dispositif d'appel de patient surveillé a changé ; et
l'indication qu'un état du dispositif d'alarme de personnel surveillé ou du dispositif d'appel de patient surveillé a changé en modifiant la manière dont le dispositif d'alarme de personnel ou le dispositif d'appel de patient est présenté sur le plan d'étage affiché.

12. Procédé selon l'une quelconque des revendications précédentes, comprenant en outre :
l'identification qu'un panneau d'observation de porte surveillée a été ouvert ; et
l'enregistrement dans la mémoire d'une indication que le panneau d'observation de porte surveillée a été ouvert.

13. Procédé selon l'une quelconque des revendications précédentes, comprenant en outre :
l'identification qu'un état d'un dispositif de surveillance de signes vitaux surveillé a changé ; et
l'indication qu'un état du dispositif de surveillance de signes vitaux surveillé a changé en modifiant la manière dont le dispositif de surveillance de signes vitaux est présenté sur le plan d'étage affiché.

14. Procédé mis en œuvre par ordinateur pour configurer un système de surveillance des portes (100), le procédé comprenant :
l'affichage (402) d'un plan d'étage (302) d'un bâtiment comprenant une ou plusieurs portes comprenant chacune un dispositif de détection de ligature ;
la réception (404) d'une saisie utilisateur indiquant une sélection d'une des une ou plusieurs portes ; et
en fonction de la saisie utilisateur, la surveillance (406) d'un état de la porte sélectionnée, la surveillance d'un état de la porte sélectionnée comprenant la surveillance du déclenchement ou non du dispositif de détection de ligature de la porte sélectionnée, et
en fonction de la saisie utilisateur, une connexion câblée étant établie entre la porte sélectionnée et un appareil informatique du système de surveillance de portes, selon lequel des données provenant de la porte sélectionnée peuvent être obtenues via une connexion câblée de bout à bout.

15. Appareil informatique configuré pour exécuter le procédé de l'une des revendications précédentes.

16. Support lisible par ordinateur comprenant des instructions qui, lorsqu'elles sont exécutées par un ordinateur, entraînent l'exécution, par l'ordinateur, du procédé selon l'une quelconque des revendications 1 à 14.

17. Programme informatique comprenant des instructions qui, lorsque le programme est exécuté par un ordinateur, entraînent l'exécution, par l'ordinateur, du procédé selon l'une quelconque des revendications 1 à 14.
